# EUROPEAN PATENT APPLICATION

(11) **EP 4 393 447 A1**
(43) Date of publication of application: **03.07.2024**
(21) Application number: 22425063.9
(22) Date of filing: 29.12.2022
(51) Int. Cl.: A61C 9/00

(54) **DENTAL DEVICE FOR SCANNING ORAL CAVITY AND RELATED FUNCTIONAL MEMBER**

(71) Applicant: Fabbri, Giacomo, 47841 Cattolica (RN) (IT)
(72) Inventor: Fabbri, Giacomo, 47841 Cattolica (RN) (IT)
(74) Representative: Petraz, Gilberto Luigi

(57) **Abstract**

Dental device (10) for scanning the oral cavity provided with at least one detection member (11), which detects a plurality of data of the oral cavity, and comprising at least one functional member (12) associated with the detection member (11) and provided with at least one cleaning nozzle (18) suitable to emit a certain fluid under pressure in correspondence with the portion of oral cavity on which the detection member (11) is oriented.

## Description

### FIELD OF THE INVENTION

The present invention concerns a dental device used to carry out a digital scan of the oral cavity, so as to detect a plurality of data relating to the shape, size and color of the patient's teeth, in order to obtain a three-dimensional digital model of the dental arches, for example for diagnostic, prosthetic or other purposes.

### BACKGROUND OF THE INVENTION

Devices provided with intra-oral optical probes, used for scanning the oral cavities of patients, are known in dental medicine.

These devices are designed to detect tens or hundreds of frames per second, then to process the data detected according to certain algorithms and to provide a more or less detailed three-dimensional image of at least part of the dental arch. With these devices it is the dentist who directs and moves the device, to scan the entire upper and/or lower arch, or parts thereof.

These known devices allow to take the impression of the dental arch without needing to resort to the usual pasty resins, from which the mold is then taken.

However, to allow the probe to detect clear images, the scanned tooth must be as dry as possible, free of intra-oral fluids, such as saliva, blood or others, so the normal practice is to use the usual suction and rinsing devices, which free the mouth from these liquids.

It is clear that this combined situation partly reduces the maneuverability of the probe, leading to the detection of frames that are not always precise and pertinent, with negative consequences on the quality and reliability of the three-dimensional workings obtained.

Furthermore, it must be considered that due to the intrinsic nature of dental medicine, the dentist usually has to operate in inconvenient conditions and in small spaces. Even considering that the probe is able to acquire many images per second, it is very frequently the case that the doctor's hand and/or the patient move, even with movements that are imperceptible yet sufficient to make the images blurred and unclear.

In the same way, to allow maneuverability and precision for the probe, the suction devices are positioned at a certain distance from the tooth or from the portion of the dental arch that is being detected, so as not to directly interfere with the detection, therefore they partly lose their effectiveness, again to the detriment of the quality of the frames detected.

In all of this, it must be taken into consideration that the patient must bear the simultaneous positioning and movement, inside the mouth, of different devices and instruments.

This aspect is even more evident in precision impressions, in which it is desired to proceed with a displacement of the gingival sulcus using a special dental technique, which provides, for example, to use retractions cords. This particular condition also puts the dentist in a situation of poor maneuverability, with a consequent worsening of the precision of the operations performed.

There is therefore a need to perfect a dental device for scanning the oral cavity, which can overcome at least one of the disadvantages of the state of the art.

To do this it is necessary to solve the technical problem of increasing the reliability and precision of the scan of the oral cavity, in most operating conditions and modes.

One purpose of the present invention is to provide a dental device for scanning the oral cavity in which the tooth, or the portion of the tooth to be scanned, is substantially free of interfering elements such as, for example, intra-oral liquids, without using third-party devices or additional equipment.

Another purpose of the present invention is to provide a dental device which can be easily maneuvered by the dentist, so as to allow maximum positioning precision, in order to detect specific and high-quality frames, and to process three-dimensional digital models as faithful as possible to the real condition.

Another purpose of the present invention is to provide a dental device which is simple and economical to produce, and which can be used and compared to traditional ones, while overcoming the disadvantages listed herein.

The Applicant has devised, tested and embodied the present invention to overcome the shortcomings of the state of the art and to obtain these and other purposes and advantages.

### SUMMARY OF THE INVENTION

The present invention is set forth and characterized in the independent claims.

The dependent claims describe other characteristics of the present invention or variants to the main inventive idea.

In accordance with the above purposes, and to resolve the technical problem disclosed above in a new and original way, also achieving considerable advantages compared to the state of the prior art, according to the present invention a dental device is provided for scanning the oral cavity of a patient, by means of at least one member for detecting a plurality of data, for example frames, of a portion or of the entire oral cavity, both upper and lower.

The frames are then processed to provide the dentist with a three-dimensional image or model of the scanned oral cavity, both for diagnostic as well as studying or prosthetic purposes.

The detection member traditionally comprises a central body manipulated by the dentist, and a detection area that the doctor selectively directs toward the various areas of the dental arch, to detect the frames and, therefore, the characteristics thereof.

In accordance with one aspect of the present invention, the dental device comprises at least one functional member, which is associated, in a removable or permanent manner, with the detection member itself, and is provided with at least one cleaning nozzle. The latter is suitable to emit a certain fluid under pressure in correspondence with the portion of the oral cavity to be detected, that is, the one on which the dentist orients the detection area of the detection member.

In this way, together with the movement of the detection device, a fluid under pressure is blown on the portion of the teeth subjected to scanning.

This solution allows to keep the scanned portion substantially free of intra-oral fluids, such as saliva, blood or suchlike, to the advantage of the quality of the frames detected and, consequently, the quality and precision of the subsequent three-dimensional digital reconstruction.

The solution according to the present invention allows maximum positioning precision of the device inside the patient's oral cavity, in order to detect specific and high quality frames, and to process three-dimensional digital models as faithful to the real condition as possible.

Advantageously, the fluid blown into the dental arch is an inert gas such as air, nitrogen or suchlike, or a mixture of gas and liquid, for example air and aqueous dust and/or disinfectant solution, depending on the different types of interventions to be performed.

Furthermore, the insufflated fluid has a drying effect, thus making the tooth less bright and less reflective to light, so as to further improve the quality of the frames acquired, in particular for devices that scan by light beam emission.

Another advantage of the solution according to the present invention is that in the zone where the teeth are attached to the gum, the insufflated fluid facilitates the displacement of the sulcus, effectively moving the gums, specifically the gingival margin, slightly away from the tooth, and allowing to reconstruct the profile of the demarcation line between them with greater precision. This is possible because, by displacing the gingival margin, it is possible to better open the gingival sulcus, improving the quality and depth of the reading of the sulcus and of the demarcation line between the tooth and the gums.

Here too, the use of additional dental instruments is not required, to the advantage of the maneuverability of the device, the quality of the detection and the comfort of both the patient as well as the dentist.

In accordance with another aspect of the present invention, the functional member comprises two cleaning nozzles, which are disposed, respectively, on opposite parts with respect to the detection member, and in particular laterally with respect to its detection area.

This advantageous solution allows for a greater and more effective cleaning and drying of the teeth involved in the scan, further improving the quality of the frames detected and, therefore, of the digital reconstructions processed.

According to some embodiments of the present invention, each of the cleaning nozzles has a substantially circular, or slotted, outlet section to better intervene in the action of displacing the gingival sulcus.

In accordance with another aspect of the present invention, the functional member comprises a circuit provided with at least one front end on which the cleaning nozzle is made. In other embodiments, the fluid circuit also has a rear end on which at least one attachment seating is made for the connection to a circuit/system for supplying the fluid.

In accordance with another aspect of the present invention, the functional member comprises at least one support body, shaped to at least partly, or almost completely, wrap the detection member, providing an aperture in correspondence with the area through which the data of the oral cavity is detected.

The support body is provided with coupling means of the removable type, which allow it to be selectively coupled to the detection member. According to some embodiments, which fall within the scope of the inventive idea of the present invention, the coupling means can provide clips or other elastic members, such as for example springs, flexible and elastic caps and films which can be slipped onto the detection member like a sheath that adheres and adapts to the shape of the latter, copying its shape, interlocking conical shapings, screw clamps, in any case of the removable type, to allow the selective removal of the support body from the detection member.

This advantageous solution of the present invention allows, on the one hand, to provide a substantially disposable support body and, on the other hand, by protecting the detection member, it reduces the need to subject the detection member itself to intense sterilization cycles.

In accordance with another aspect of the present invention, the fluid circuit, with the corresponding nozzles, is integrated in the support body.

According to another aspect, the present invention also concerns only the functional member, whether it is made independently to be selectively associated, either permanently or removably, with the detection member, or whether it is made in one piece, that is, integrated in a single body, with the latter.

In accordance with another aspect of the present invention, there is also provided a method for scanning the oral cavity comprising at least a step of detecting a plurality of data of the oral cavity by means of the at least one detection member, a step of emitting a fluid under pressure in correspondence with the portion of the oral cavity to be detected on which the detection member is oriented, by means of a functional member associated with the detection member and provided with at least one cleaning nozzle suitable to emit the fluid under pressure.

### DESCRIPTION OF THE DRAWINGS

These and other aspects, characteristics and advantages of the present invention will become apparent from the following description of some embodiments, given as a non-restrictive example with reference to the attached drawings wherein:
- fig. 1 is a schematic three-dimensional view of a first embodiment of a dental device according to the present invention;
- fig. 2 shows a front view, from below, of the embodiment of fig. 1;
- the fig. 3 shows a lateral view of the embodiment of fig. 1;
- fig. 4 is a schematic three-dimensional view of a second embodiment of a dental device according to the present invention;
- fig. 5 shows a front view, from below, of the embodiment of fig. 4;
- the fig. 6 shows a lateral view of the embodiment of fig. 4.

We must clarify that in the present description the phraseology and terminology used, as well as the figures in the attached drawings also as described, have the sole function of better illustrating and explaining the present invention, their function being to provide a non-limiting example of the invention itself, since the scope of protection is defined by the claims.

To facilitate comprehension, the same reference numbers have been used, where possible, to identify identical common elements in the drawings. It is understood that elements and characteristics of one embodiment can be conveniently combined or incorporated into other embodiments without further clarifications.

### DESCRIPTION OF SOME EMBODIMENTS OF THE PRESENT INVENTION

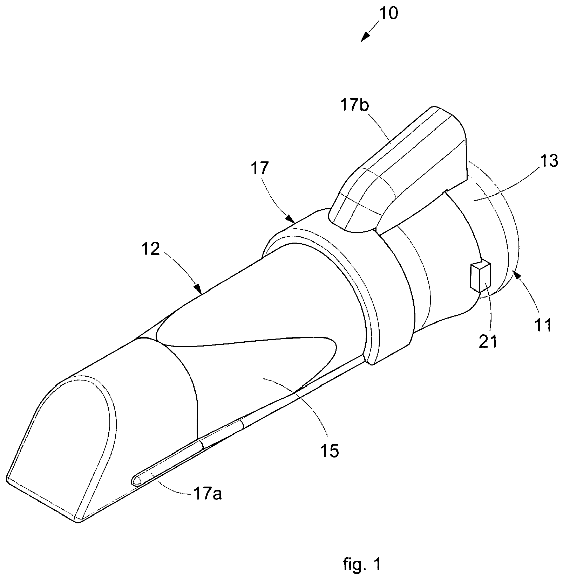

With reference to fig. 1, a dental device 10 according to the present invention substantially comprises a detection member, or optical probe 11, and a functional member 12 associated with the probe 11.

For descriptive purposes only, the optical probe 11 is of the intra-oral type, normally used in the medical-dental field to scan the oral cavity, or part of it, in order to detect a plurality of data functional for the digital reconstruction of a three-dimensional model, the latter usable for diagnostic, study, prosthetic, or other purposes.

In the description and in the schematic illustrations of the embodiments of the device 10 according to the present invention which will be described hereafter, specific and/or manufacturing details of the probe 11 will not be given, since the latter can be freely selected from those available on the market. The person of skill in the art is perfectly capable of adapting the functional member 12 described below to the geometry of the probe 11, which may have minimal manufacturing differences from one producer to another.

For descriptive purposes only, the probe 11 traditionally comprises a central body 13 and a detection area 14, for example optical, to capture tens or hundreds of frames per second of the teeth that the dentist chooses to detect, in order to recreate the entire, or part of the, upper and/or lower arch, as needed.

In the embodiment shown in figs. 1, 2 and 3, the functional member 12 has a support body 15, in this specific case shaped to almost completely wrap the central body 13 of the probe 11.

By way of example, in the solution shown, the mating coupling between the support body 15 and the corresponding central body 13 defines an integral coupling between the functional member 12 and the probe 11. This guarantees the operative coupling between the two elements 11 and 12 during the manipulation and detection steps.

According to an advantageous variant, shown only schematically in the attached drawings, the coupling between the probe 11 and the functional member 12 can be actuated, for example, by means of a pair of elastic clips 21, made on opposite parts on the support body 15 and suitable to cooperate with corresponding shapings of the probe 11.

According to other variants of the present invention, the coupling between the two elements 11 and 12 can be actuated by means of a radial band which radially tightens part of the functional member 12 onto the probe 11, or screw systems could be provided, either for direct locking or radial constriction of the functional member 12 onto the probe 11.

It must be stated that these or other coupling solutions can be chosen from those which allow a selective removability of the functional member 12 with respect to the probe 11, for example in disposable embodiments, or which guarantee an irremovable coupling between the two. According to another variant of the inventive idea according to the present invention, it is not excluded that the functional member 12 can be made in one piece with the probe 11 and therefore be completely integrated with it.

Returning to the specific description of the embodiment shown in figs. 1, 2 and 3, the support body 15 is provided at the lower part with an aperture 16, made in correspondence with the detection area 14 of the probe 11, so as to guarantee maximum frame detection functionality.

Furthermore, according to the present invention, a fluid circuit 17 is made on the support body 15, extending from a first end 17a made in correspondence with, or at least in proximity to, the aperture 16 and a second end 17b made on the opposite part and facing toward the rear part of the central body 13 of the probe 11.

According to one variant, not shown in the drawings, the fluid circuit 17 can be applied, in a fixed or removable manner, to the support body 15. In any case, the first end 17a comprises, in this case, a pair of circular apertures 18 facing, during use, toward the portion of the dental arch being detected.

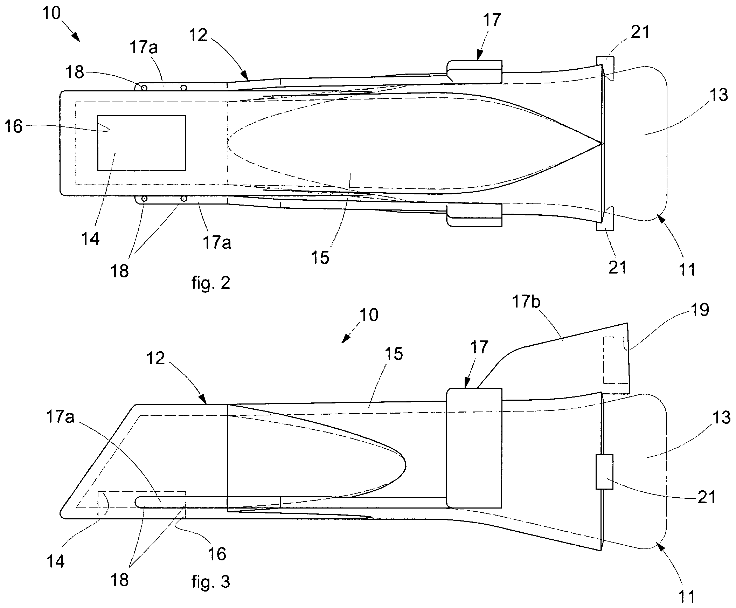

With particular reference to fig. 2, the fluid circuit 17 comprises two first ends 17a, each provided with corresponding openings 18, and disposed on opposite parts with respect to the aperture 16.

According to some variants, not shown in the drawings, the number of openings 18 and their disposition can be different, and they can also have a slotted, rather than circular, conformation.

The second end 17b, on the other hand, provides a rapid attachment seating 19 (fig. 3), so that the fluid circuit 17 can be selectively associated with a corresponding system for supplying a fluid under pressure, in this case air. The system for supplying a fluid under pressure, since it is of a substantially known type, is not shown in the attached drawings.

It is clear that, depending on the types of intervention or operational needs, instead of air, another fluid can be supplied under pressure, both gaseous and liquid, or a combination of the two, as well as a selective alternation between gas and liquid.

Therefore, once connected to the system, the circuit 17 allows an insufflation under pressure of air toward the portion of the dental arch which is gradually detected through the detection area 14.

According to a variant not shown, the circuit 17 can provide one or more selection members, such as for example one or more valves, which allow the dentist to command the insufflation of the fluid through one or other of the openings 18 provided at the first end 17a, as well as choose whether to insufflate one or the other fluid through one and/or the other of the openings 18.

As previously described, the insufflation of the fluid during the steps of detecting the dental arch leads to considerable advantages both in terms of quality and reliability of the detection performed, and in terms of maneuverability of the device 10 according to the present invention, as well as in terms of comfort for the patient.

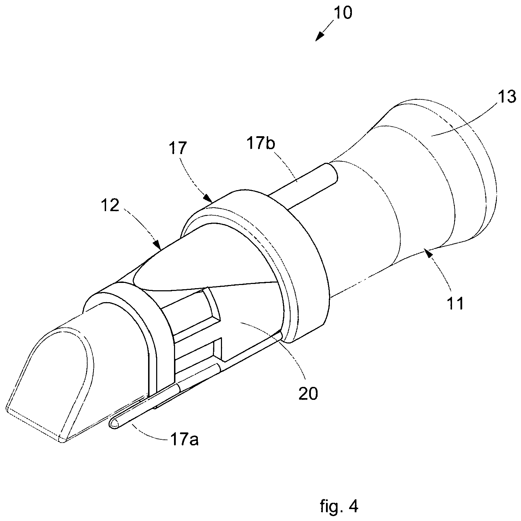

In the embodiment shown in figs. 4, 5 and 6, the functional member 12 has a support body 20 shaped to partly wrap the central body 13 of the probe 11.

Unlike the previous embodiment, the support body 20 is substantially lightened, cooperating only with a limited portion of the probe 11.

Also in this embodiment, different coupling solutions as described above can be provided, either with clips or other elastic members, as well as flexible and elastic caps and films such as for example a sheath that adheres and adapts to the shape of the probe 11, or chosen from those that allow a selective removability of the functional member 12 with respect to the probe 11, for example in disposable embodiments, or even from those that guarantee an irremovable coupling between the two. Also in this last embodiment, it is not excluded that the functional member 12 can be made in one piece, that is, in a single body, with the probe 11, and therefore be completely integrated with it.

In particular, in this second embodiment the support body 20 is concentrated in a grip zone of the probe 11, so as to promote the manipulation conditions.

Advantageously, the support body 20, but also the body 15 previously described, has/have an ergonomic shape on its/their external surface, so as to promote the conditions for gripping and manipulating the probe 11, again in order to improve the quality of the detection performed.

Also in this embodiment, a fluid circuit 17 is made on the support body 20, extending between a pair of first ends 17a and a second end 17b.

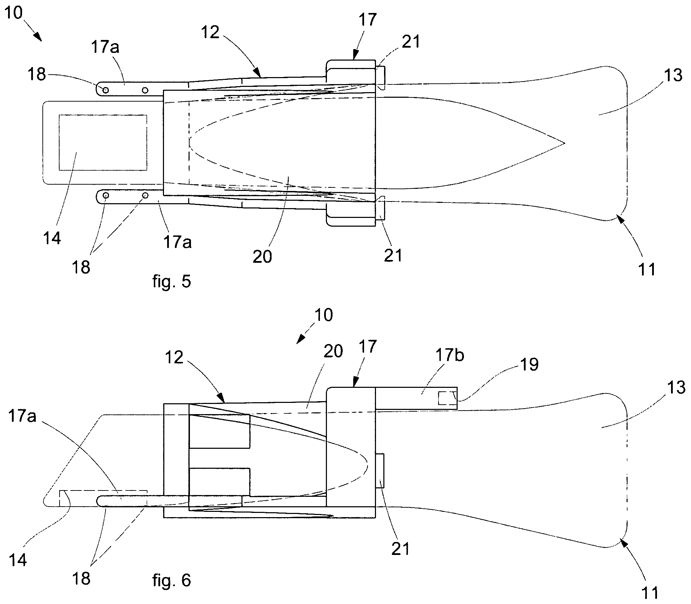

The first ends 17a are provided, respectively, with the openings 18 disposed on opposite parts with respect to the aperture 16, while the second end 17b provides the rapid attachment seating 19 (fig. 6) for the system for supplying the fluid under pressure.

It is clear that modifications and/or additions of parts may be made to the device 10 and to the functional member 12 as described heretofore, without departing from the field and scope of the present invention, as defined by the claims.

It is also clear that, although the present invention has been described with reference to some specific examples, a person of skill in the art shall certainly be able to achieve many other equivalent forms of dental device for scanning the oral cavity and corresponding functional member, having the characteristics as set forth in the claims and hence all coming within the field of protection defined thereby.

In the following claims, the sole purpose of the references in brackets is to facilitate their reading and they must not be considered as restrictive factors with regard to the field of protection defined by the same claims.

## Claims

1. Dental device (10) for scanning the oral cavity provided with at least one detection member (11) for detecting a plurality of data of said oral cavity, **characterized in that** it comprises at least one functional member (12) associated with said detection member (11) and provided with at least one cleaning nozzle (18) suitable to emit a fluid under pressure in correspondence with the portion of said oral cavity to be detected.

2. Dental device (10) as in claim 1, **characterized in that** said functional member (12) comprises at least one pair of cleaning nozzles (18) disposed, respectively, on opposite parts with respect to said detection member (11).

3. Dental device (10) as in claim 2, **characterized in that** each of said cleaning nozzles (18) has a substantially circular outlet section.

4. Dental device (10) as in claim 2, **characterized in that** each of said cleaning nozzles (18) has a substantially slotted outlet section.

5. Dental device (10) as in any claim hereinbefore, **characterized in that** said functional member (12) comprises a fluid circuit (17), being provided with at least one first end (17a) on which said at least one cleaning nozzle (18) is made.

6. Dental device (10) as in claim 5, **characterized in that** said fluid circuit (17) is provided with at least one second end on which at least one attachment seating (19) is made for a circuit/system for supplying said fluid.

7. Dental device (10) as in any claim hereinbefore, **characterized in that** said functional member (12) comprises at least one support body (15), shaped to at least partly wrap said detection member (11) and provided with coupling means (21) of the removable type.

8. Dental device (10) as in claim 7, **characterized in that** said support body (15) is shaped to almost completely wrap at least one front portion of said detection member (11).

9. Dental device (10) as in claim 8, **characterized in that** said support body (15) comprises at least one aperture (16) made in correspondence with a detection area (14) of said detection member (11).

10. Dental device (10) as in claims 5 and 7, **characterized in that** said fluid circuit (17) is integrated in said support body (15).

11. Dental device (10), as in any claim hereinbefore, **characterized in that** said functional member (12) is made in one piece with said detection member (11).

12. Functional member (12) for a dental device (10) provided with at least one detection member (11) for detecting a plurality of data of an oral cavity, **characterized in that** it is associated with said detection member (11) and it is provided with at least one cleaning nozzle (18) suitable to emit a fluid under pressure in correspondence with the portion of said oral cavity to be detected.

13. Functional member (12) as in claim 12, **characterized in that** it comprises at least one support body (15), shaped to at least partly wrap said detection member (11).

14. Functional member (12) as in claim 12 or 13, **characterized in that** it is made in one piece with said detection member (11).

15. Method for scanning the oral cavity comprising at least a step of detecting a plurality of data of said oral cavity by means of at least one detection member (11), **characterized in that** it comprises at least a step of emitting a fluid under pressure in correspondence with the portion of said oral cavity on which said detection member (11) is oriented, by means of a functional member (12) associated with said detection member (11) and provided with at least one cleaning nozzle (18) suitable to emit said fluid under pressure.
